# EUROPEAN PATENT APPLICATION

(11) **EP 0 778 466 A1**
(43) Date of publication of application: **11.06.1997**
(21) Application number: 96917718.7
(22) Date of filing: 18.06.1996
(51) Int. Cl.: G01N 33/569

(54) **ANTIBODY-SENSITIZED LATEX FOR DENITRIFYING BACTERIA AND METHOD OF DETECTING THE BACTERIA**

(30) Priority: 19.06.1995 JP 175398/95
(71) Applicant: KABUSHIKI KAISHA YAKULT HONSHA, Minato-ku Tokyo 105 (JP); Atobe, Hisao, Tachikawa-shi, Tokyo 190 (JP)
(72) Inventor: ATOBE, Hisao, Tachikawa-shi, Tokyo 190 (JP); OHMURA, Hiroshi, Minato-ku, Tokyo 105 (JP); OKUMURA, Takekazu, Minato-ku, Tokyo 105 (JP); NAGAI, Fumiko, Minato-ku, Tokyo 105 (JP); WADA, Mitsuyo, Minato-ku, Tokyo 105 (JP); YAGISHITA, Masami, Nagareyama-shi, Chiba 270-01 (JP)
(74) Representative: Baillie, Iain Cameron
(86) International application number: JP9601674
(87) International publication number: WO9700448

(57) **Abstract**

The present invention provides an antibody-sensitized latex for use in detecting conveniently denitrifying bacteria being present in activated sludge, water or soil, and a method of detecting the denitrifying bacteria by using said antibody-sensitized latex. The present invention relates to an antibody-sensitized latex used for detecting denitrifying bacteria, comprising latex particles and an antibody, wherein the antibody recognizes the denitrifying bacteria specifically and is adsorbed on the latex particles. As the latex particles, those having a high specific gravity of about 1.5 g/cc and an average particle diameter of about 1.0 µm are used. In a conventional method for detecting denitrifying bacteria, a long period of time and technical expertise have been required; however, according to the present invention, the detection and determination of the denitrifying bacteria can be performed easily and conveniently. Further, it serves to establish a method for identifying active target bacteria being present in activated sludge, water or soil and measuring the number of the bacteria rapidly and properly by employing one or optionally plural kinds of antibodies specific for the denitrifying bacteria.

## Description

### Technical Field

The present invention relates to an antibody-sensitized latex used for detecting conveniently denitrifying bacteria being present in activated sludge, water or soil, and a method of detecting denitrifying bacteria by using said antibody-sensitized latex; more specifically, the present invention relates to an antibody-sensitized latex, which facilitates the detection and determination of denitrifying bacteria which has required a long period of time and technical expertise and further serves to establish a method for controlling properly active target microorganisms present in activated sludge, water or soil, and a method of detecting denitrifying bacteria.

### Technical Background

As a method for detecting denitrifying bacteria being present in activated sludge or soil has been known a method for detecting denitrifying bacteria according to the presence of nitrogen-containing gas produced by a sample of the bacteria collected and cultured for the period of 10 to 14 days; target bacteria (denitrifying bacteria) having an activity (to produce nitrogen gas and nitrous oxide gas due to the reduction of nitric acid and nitrous acid) being present in activated sludge or soil have been controlled by utilizing said method.

However, said method has required technical expertise for the detection and measurement of denitrifying bacteria and has taken a long period of time, and hence it has been hard to take a rapid countermeasure for controlling target bacteria according to the results of the measurement.

In addition, denitrifying bacteria often include strains lacking in reductase to reduce nitrous oxide, and in such a case, the presence of gas produced cannot be confirmed since nitrous oxide has a large solubility, and precise judgment of it has been hard in such samples.

On the other hand, the present inventors have developed a method of detecting nitrate or nitrite bacteria using an antibody and has provided a method of detecting rapidly the nitrate or nitrite bacteria by using the method (Official Gazette of Japanese Laid-Open Patent Publication No. 5-322896/1993).

In addition, they have provided a method of detecting nitrate or nitrite bacteria readily by using an antibody-sensitized latex comprising said antibody adsorbed on latex particles (Japanese Patent Application No. 6-76336/1994).

Under these circumstances, the present inventors have engaged in assiduous studies with a view to developing a method of detecting conveniently and rapidly denitrifying bacteria being present in activated sludge, water or soil.

As a result, the present inventors have found that denitrifying bacteria can be detected and determined conveniently and precisely by preparing an antibody specific for denitrifying bacteria and employing an antibody-sensitized latex comprising said antibody adsorbed on latex particles, which has led to the accomplishment of the present invention.

### Summary of the Invention

The present invention provides an antibody-sensitized latex for use in detecting conveniently denitrifying bacteria being present in activated sludge, water or soil, and a method of detecting the denitrifying bacteria by using said antibody-sensitized latex.

The present invention relates to an antibody-sensitized latex used for detecting denitrifying bacteria, comprising latex particles and an antibody, wherein the antibody recognizes the denitrifying bacteria specifically and is adsorbed on the latex particles. As the latex particles, those having a high specific gravity of about 1.5 g/cc and an average particle diameter of about 1.0 µm are used.

In a conventional method for detecting denitrifying bacteria, a long period of time and technical expertise have been required; however, according to the present invention, the detection and determination of the denitrifying bacteria can be performed easily and conveniently.

Further, it serves to establish a method for identifying active target bacteria being present in activated sludge, water or soil and measuring the number of the bacteria rapidly and properly by employing one or optionally plural kinds of antibodies specific for the denitrifying bacteria.

### Disclosure of the Invention

It is an object of the present invention to provide a novel antibody-sensitized latex used for detecting denitrifying bacteria, which facilitates the detection of denitrifying bacteria being present in activated sludge, water or soil.

It is another object of the present invention to establish a method for detecting and determining conveniently denitrifying bacteria being present in activated sludge, water or soil by utilizing the above antibody-sensitized latex for detection.

The present invention dissolving the above problems relates to an antibody-sensitized latex for denitrifying bacteria characterized by comprising latex particles and an antibody, wherein the antibody is specific for the denitrifying bacteria and adsorbed on the latex particles.

Another embodiment of the present invention is an antibody-sensitized latex for denitrifying bacteria characterized by employing latex particles having a high specific gravity of around 1.5 g/cc.

Still another embodiment of the present invention is an antibody-sensitized latex for denitrifying bacteria characterized by employing latex particles having an average particle diameter of around 1.0 µm.

Moreover, still another embodiment of the present invention is a method of detecting denitrifying bacteria characterized by employing the above antibody-sensitized latex for denitrifying bacteria.

Denitrifying bacteria in the present invention are microorganisms having a denitrifying activity, namely, a function of producing nitrogen gas and nitrous oxide gas due to the reduction of nitric acid and nitrous acid, and some species of them have been known. In the method of detecting denitrifying bacteria employing the antibody of the present invention, said antibody is generally specific for the species; hence, it is a principle to prepare antibodies to all denitrifying bacteria and employing them; however, it is not always necessary to employ all antibodies in the measurement of denitrifying bacteria being present in ordinary activated sludge or soil; for example, the object can be accomplished sufficiently by employing antibodies to standard strains selected as preferable ones in the present invention in combination, as will be shown below.

In the present invention, Agrobacterium radiobacter; IFO 13532T strain, Alcaligenes denitrificans; IFO 15125T strain, Bacillus licheniformis; IFO 12200T strain, Flavobacterium sp.; IFO 15128 strain, Flavobacterium sp.; IFO 15147 strain, Paracoccus denitrificans; IFO 14907 strain, Pseudomonas stutzeri; IFO 14165T strain, Pseudomonas fluorescens; IFO 14160T strain, Pseudomonas picketti; JCM 5969T strain, Thiobacillus denitrificans; JCM 3870 strain, Pseudomonas aeruginosa; IFO 12689T strain and Alcaligenes faecalis; IFO 13111T strain are used preferably as standard strains of denitrifying bacteria for preparing antibodies; however, it goes without saying that antibodies can be prepared similarly by using strains of other denitrifying bacteria. As described above, the present invention is performed preferably by combining the antibodies to the above standard strains properly; it is also possible, however, to combine antibodies to strains of other denitrifying bacteria properly.

Antibodies specific for these bacteria are used by preparing an antiserum according to a known method (e.g., method described in the Official Gazette of Japanese Laid-Open Patent Publication No. 5-322896/1993) and further purifying it by a proper means of purification such as the protein A column kit.

That is, in order to prepare an antibody specific for the above denitrifying bacteria, first of all, each bacterium is cultured with a suitable liquid medium, sterilized if necessary, and collected to obtain an antigen. Subsequently, the antigen is injected, for example, into the ear vein of a rabbit, and after confirming the increase of the antibody titer, blood is collected therefrom, subjected to a suitable treatment such as centrifugal separation and inactivated at 56 °C to obtain an antiserum.

Further, it is purified with a suitable means of purification such as the protein A column kit to obtain an antibody.

Subsequently, latex particles of a carrier to adsorb the above specific antibody and a method of preparing a sensitized latex can be performed according to the method reported by the present inventors (Japanese Patent Application No. 6-76336/1994).

That is, as latex particles, those having a high specific gravity of about 1.0 to 1.5 g/cc, preferably around 1.5 g/cc, and an average particle diameter of about 0.8 to 2.2 µm, preferably around 1.0 µm can be employed suitably.

As such latex particles, for example, commercially available products such as Bactlatex 0.81 (manufactured by Difco, average particle diameter: 0.81 µm, specific gravity: 1.0 g/cc), and H0901, H0902 and H2002 (manufactured by Nihon Gousei Gom, JP, average particle diameter/specific gravity: 0.94 µm/1.5 g/cc, 0.98 µ m/1.5 g/cc and 2.22 µm/1.5 g/cc, respectively) can be mentioned; however, other products with the same effect can be utilized irrespective of kind.

It has been revealed according to the examination by the present inventors, as will be described later, that in the case of those sensitized to latex particles having a specific gravity of around 1.0 g/cc, agglutination images can be judged sufficiently according to a method for observing them with a microscope (microscope latex method), but that it is difficult to judge agglutination images according to a method for observing them with the naked eye (microtiter method). In contrast, in those sensitized to latex particles having a high specific gravity of around 1.5 g/cc, agglutinated latex particles precipitate rapidly and can be judged with the naked eye easily and precisely; hence latex particles having a high specific gravity of around 1.5 g/cc are used preferably in the present invention.

As will be described later, a sensitized latex having a high sensitivity can be obtained by employing latex particles having an average particle diameter of around 1.0 µm; hence latex particles having an average particle diameter of around 1.0 µm are used preferably in the present invention.

An antibody-sensitized latex can be obtained by mixing a suspension of latex particles diluted with a proper buffer and a buffer solution of an antibody; as a buffer can be utilized an ammonium chloride buffered-saline solution, a PBS buffer and a glycine buffered-saline solution; of them, the glycine buffered-saline solution is used preferably from the viewpoints of the agglutination properties of a sensitized latex and the difficulty in causing self-agglutination of it.

The concentration of an antibody-sensitized latex is adjusted to from 0.05 to 1.0 %, preferably from 0.1 to 0.5 %, and more preferably around 0.25 %.

The concentration of an antibody protein in sensitization is preferably from 0.05 to 0.3 %; otherwise, it tends to be difficult due to the deterioration of the sensitivity of an antibody-sensitized latex and the self-agglutination of it to distinguish between positivity and negativity thereof.

A latex is sensitized at a temperature within the range of 0 to 60 °C ; if the latex is sensitized at room temperature or at a temperature slightly higher than it (to 40 °C ), a sensitized latex with a high sensitivity can be obtained.

Denitrifying bacteria are detected and the number of the bacteria is measured by using such sensitized latices, mixing a sample livid diluted to a proper grade and a sensitized-latex solution and confirming the degree of dilution capable of observing agglutination images, on the other band, performing a mixing test employing a standard sample of a bacterium liquid, and calculating the number of the bacteria in comparison with the result thereof.

As a specific method in the present invention can be employed the microtiter method capable of judging agglutination images with the naked eye simply. That is, they can be observed and judged with the naked eye or with a magnifier of about 10 magnifications after the steps of sampling an antigen or a sample solution diluted gradually with a buffer onto a U-shaped microtiter plate, pouring an antibody-sensitized latex into each well of the plate and leaving it as it is at room temperature for 3 to 15 hours.

Specifically, agglutination images of each well are judged by deeming agglutination images in the case of a buffer alone as negative, and the number of bacteria is calculated from the dilution rate of the well to be tested showing positive agglutination images and the result of an agglutination test employing a standard antigen.

It goes without saying that, in the present invention, in addition to the above, various methods for detection and determination employing an antibody-sensitized latex with an antibody of denitrifying bacteria absorbed on it, such as a method for judging the presence of agglutination images by mixing an antibody-sensitized latex according to the present invention and a sample to be tested on a slide glass and observing it with optical microscope (microscope latex method) and the like can be employed.

The existence of latex particles as a carrier capable of adsorbing an antibody has been known; however, the actual use thereof for the detection and identification of denitrifying bacteria has not been reported, and the efficiency thereof has been investigated by the present inventors for the first time.

In particular, the fact that it is possible to establish a method for identifying target bacteria having an activity (to produce nitrogen gas and nitrous oxide gas due to the reduction of nitric acid and nitrous acid) being present in activated sludge, water or soil and controlling the number of bacteria rapidly and properly has been found by the present inventors for the first time.

### Best Mode for Carrying out the Invention

Hereunder, the present invention will be described in more detail according to Example, but the present invention is restricted to said Example by no means.

### Example

### 1. Preparation of a Bacterial Cell Solution for an Antigen

As denitrifying bacteria were employed Agrobacterium radiobacter (IFO 13532T strain), Alcaligenes denitrificans (IFO 15125T strain), Bacillus licheniformis (IFO 12200T strain), Flavobacterium sp. (IFO 15128T strain), Flavobacterium sp. (IFO 15147T strain), Paracoccus denitrificans (IFO 14907T strain), Pseudomonas stutzeri (IFO 14165T strain), Pseudomonas fluorescens (IFO 14160T strain) and Pseudomonas picketti (JCM 5969T strain); said denitrifying bacteria were cultured under shaking (medium: 100 ml) in a Sakaguchi flask under the culture conditions of Table 1.

Thiobacillus denitrificans was subjected to stationary culture (medium: 1 liter) under the culture conditions of Table 1.

Regarding the composition of the mediums, the composition of Medium 1 in Table 1 comprises 10 g of polypeptone, 2 g of a yeast extract and 5 g of sodium chloride; the above compounds were dissolved in 1 l of distilled water to adjust a pH to 7, autoclaved and subjected to use.

Medium 2 is an LB medium and the composition thereof comprises 10 g of trypton, 5 g of a yeast extract, 5 g of sodium chloride, 1 g of glucose, 10 ml of 1 M Toris and 1 ml of 1 M magnesium sulfate heptahydrate; the above compounds were dissolved in 1 l of distilled water to adjust a pH to 7, autoclaved and subjected to use.

Medium 3 is an NB medium and the composition thereof comprises 10 g of a meat extract, 10 g of polypeptone and 3 g of sodium chloride; the above compounds were dissolved in 1 l of distilled water to adjust a pH to 7, autoclaved and subjected to use.

Medium 4 is an SCY modified medium and the composition thereof comprises 1 g of sucrose, 0.75 g of Bactokajiton (phonetic), 0.25 of a yeast extract, 0.25 g of trypsin digestive peptone, 0.0004 g of vitamin B₁ and 0.00001 g of vitamin B₁₂; the above compounds were dissolved in 1 l of distilled water to adjust a pH to 7, autoclaved and subjected to use.

Medium 5 is a TSBYE medium and the composition thereof comprises 10 g of a trypsin digestive soybean extract, 10 g of a yeast extract and 3 g of sodium chloride; the above compounds were dissolved in 1 l of distilled water to adjust a pH to 7, autoclaved and subjected to use.

Medium 6 is an S8 medium and the composition thereof comprises 1.8 g of potassium di hydrogenphosphate, 1.2 g of disodium hydrogenphosphate, 0.1 g of ammonium sulfate, 5 g of potassium nitrate, 0.1 g of magnesium sulfate heptahydrate, 0.03 g of ferric chloride hexahydrate, 0.03 g of manganese sulfate pentahydrate, 0.04 g of calcium chloride dihydrate and 0.5 g of sodium carbonate; the above compounds were dissolved in 0.9 l of distilled water to adjust a pH to 7 and autoclaved, and 100 ml of sodium thiosulfate were added thereto to make 1 l of a product to be used.

The culture was terminated at the time when the growth of bacteria peaked out; the reaction mixture was condensed according to centrifugal separation, washed with a 0.85 % physiological saline, mixed with on equivalent amount of a 0.6 % formaldehyde solution, left to stand at 37 °C overnight to sterilize bacteria, washed with a 0.85 % physiological saline and adjusted with a physiological saline to have a concentration of absorbance (OD550) ≒ 0.5 to obtain a sample antigen.

**Table 1**

| Name of bacterium | Strain No. | Medium | Culture time |
|---|---|---|---|
| Agrobacterium radiobacter | IFO 13532T | 1 | 2-3 days |
| Alcaligenes denitrificans | IFO 15125T | 2 | 2-3 days |
| Bacillus licheniformis | IFO 12200T | 3 | 2-3 days |
| Flavobacterium sp. | IFO 15128 | 4 | 3-4 days |
| Flavobacterium sp. | IFO 15147 | 4 | 5-7 days |
| Paracoccus denitrificans | IFO 14907 | 2 | 2-3 days |
| Pseudomonas stutzeri | IFO 14165T | 2 | 2-3 days |
| Pseudomonas fluorescens | IFO 14160T | 5 | 2-3 days |
| Pseudomonas picketti | JCM 5969T | 5 | 2-3 days |
| Thiobacillus denitrificans | JCM 3870 | 6 | 10-14 days |

### 2. Preparation of a Corresponding Anti-rabbit Serum

Employing two KBL:JW (Japanese white species) rabbits, the antigen solution of denitrifying bacteria prepared by adjusting the antigen concentration according to turbidity (CD660 ≒ 0.5) was injected into the ear vein of each in an amount of 0.5 ml. The antigen solution was injected into it additionally from 4 to 6 times every 7 to 10 days, and from the third time on, an antibody titer of it was measured according to an enzyme antibody method.

When an increase of the antibody titer was not observed, blood was collected from the heart thereof. The blood was inactivated at 56 °C to obtain an antiserum corresponding to the denitrifying bacteria.

### 3. Test regarding the Specificity of Each Antiserum

The specificity to antigens of 10 kinds of antiserums prepared to denitrifying bacteria was compared by using a coloring reaction according to the enzyme-linked immunosorbent assay (ELISA).

That is, a solution to be examined containing denitrifying bacteria (carbonic acid-bicarbonic acid buffer solution) was poured to a microtiter plate (100 µl/well), left to stand at 5 °C for 16 hours to be adsorbed. The resultant solution was washed with a phosphoric acid buffer containing 0.05 % Triton X-100 (Buffer 1) and subsequently an antiserum prepared in the above Example (phosphoric acid buffer containing 0.05 % Triton X-100 and 10 % BSA (Buffer 2)) was added thereto in an amount of 90 µl/well, and the resultant mixture was left to stand at 37 °C for 1.5 hours. The mixture was washed with Buffer 1 and subsequently peroxidase-labelled anti-rabbit-goat IgG as an enzyme-labelled antibody was added thereto in an amount of 100 µ l/well, and the resultant product was left to stand at 37 °C for 1.5 hours under a dark condition. The resultant product was washed with Buffer 1 and subsequently a citric acid buffer solution of o-phenylenediamine containing hydrogen peroxide as a substrate was added thereto in an amount of 100 µ l/well, and the mixture was left to stand at 37 °C for 10 minutes under a dark condition to allow to develop color of it. To the resultant product was added 2.5 M sulfuric acid in an amount of 50 µl/well, and the reaction was terminated to measure absorbance of it at 492 nm.

An index shows specificity to other antigens in the case that the reciprocal of a dilution rate (ELISA value) showing absorbance (OD492 = 0.5) according to a coloring reaction applying the ELISA of antiserum prepared to an antigen is 100 %. The results are shown in Tables 2 to 11.

**Table 2**

| Specificity of Agrobacterium radiobacter (13532T) antiserum | | |
|---|---|---|
| Name of bacterium | ELISA value | Specificity (%) |
| Agrobacterium radiobacter | 336.9 | 100.0 |
| Alcaligenes denitrificans | 0.460 | 0.137 |
| Bacillus licheniformis | 0.220 | 0.065 |
| Flavobacterium sp. | 5.800 | 1.721 |
| Flavobacterium sp. | 0.690 | 0.205 |
| Paracoccus denitrificans | 1.730 | 0.513 |
| Pseudomonas stutzeri | 1.420 | 0.421 |
| Pseudomonas fluorescens | 0.600 | 0.178 |
| Pseudomonas picketti | 5.740 | 1.704 |
| Thiobacillus denitrificans | 3.380 | 1.003 |
| ELISA value: (Dilution rate showing absorbance (OD492) = 0.5 of an antibody prepared to an antigen/dilution rate showing absorbance (OD492) = 0.5 of the antibody before immunization) x 100 Specificity: Cross rate with other antigens in the case that the ELISA value of the antibody prepared to the antigen is 100 | | |

**Table 3**

| Specificity of Alcaligenes denitrificans (15125T) antiserum | | |
|---|---|---|
| Name of bacterium | ELISA value | Specificity (%) |
| Agrobacterium radiobacter | 76.07 | 5.712 |
| Alcaligenes denitrificans | 1332 | 100.0 |
| Bacillus licheniformis | 4.640 | 0.348 |
| Flavobacterium sp. | 56.10 | 4.213 |
| Flavobacterium sp. | 20.40 | 1.532 |
| Paracoccus denitrificans | 7.890 | 0.592 |
| Pseudomonas stutzeri | 24.14 | 1.813 |
| Pseudomonas fluorescens | 12.61 | 0.947 |
| Pseudomonas picketti | 21.42 | 1.608 |
| Thiobacillus denitrificans | 21.01 | 1.578 |
| ELISA value: (Dilution rate showing absorbance (OD492) = 0.5 of an antibody prepared to an antigen/dilution rate showing absorbance (OD492) = 0.5 of the antibody before immunization) x 100 Specificity: Cross rate with other antigens in the case that the ELISA value of the antibody prepared to the antigen is 100 | | |

**Table 4**

| Specificity of Bacillus licheniformis (12200T) antiserum | | |
|---|---|---|
| Name of bacterium | ELISA value | Specificity (%) |
| Agrobacterium radiobacter | 30.77 | 40.03 |
| Alcaligenes denitrificans | 21.64 | 28.16 |
| Bacillus licheniformis | 76.86 | 100.0 |
| Flavobacterium sp. | 44.90 | 58.42 |
| Flavobacterium sp. | 5.860 | 7.624 |
| Paracoccus denitrificans | 3.510 | 4.567 |
| Pseudomonas stutzeri | 9.380 | 12.20 |
| Pseudomonas fluorescens | 44.85 | 58.35 |
| Pseudomonas picketti | 21.00 | 27.32 |
| Thiobacillus denitrificans | 6.520 | 8.483 |
| ELISA value: (Dilution rate showing absorbance (OD492) = 0.5 of an antibody prepared to an antigen/dilution rate showing absorbance (OD492) = 0.5 of the antibody before immunization) x 100 Specificity: Cross rate with other antigens in the case that the ELISA value of the antibody prepared to the antigen is 100 | | |

**Table 5**

| Specificity of Flavobacterium sp. (15128) antiserum | | |
|---|---|---|
| Name of bacterium | ELISA value | Specificity (%) |
| Agrobacterium radiobacter | 2.970 | 2.525 |
| Alcaligenes denitrificans | 0.280 | 0.238 |
| Bacillus licheniformis | 0.150 | 0.128 |
| Flavobacterium sp. | 117.6 | 100.0 |
| Flavobacterium sp. | 115.0 | 97.76 |
| Paracoccus denitrificans | 0.240 | 0.204 |
| Pseudomonas stutzeri | 0.750 | 0.638 |
| Pseudomonas fluorescens | 0.470 | 0.400 |
| Pseudomonas picketti | 1.570 | 1.335 |
| Thiobacillus denitrificans | 0.310 | 0.264 |
| ELISA value: (Dilution rate showing absorbance (OD492) = 0.5 of an antibody prepared to an antigen/dilution rate showing absorbance (OD492) = 0.5 of the antibody before immunization) x 100 Specificity: Cross rate with other antigens in the case that the ELISA value of the antibody prepared to the antigen is 100 | | |

**Table 6**

| Specificity of Flavobacterium sp. (15147) antiserum | | |
|---|---|---|
| Name of bacterium | ELISA value | Specificity (%) |
| Agrobacterium radiobacter | 5.540 | 2.652 |
| Alcaligenes denitrificans | 2.140 | 1.024 |
| Bacillus licheniformis | 0.300 | 0.144 |
| Flavobacterium sp. | 868.9 | 415.9 |
| Flavobacterium sp. | 208.9 | 100.0 |
| Paracoccus denitrificans | 0.780 | 0.373 |
| Pseudomonas stutzeri | 2.000 | 0.957 |
| Pseudomonas fluorescens | 2.930 | 1.403 |
| Pseudomonas picketti | 5.750 | 2.752 |
| Thiobacillus denitrificans | 1.550 | 0.742 |
| ELISA value: (Dilution rate showing absorbance (OD492) = 0.5 of an antibody prepared to an antigen/dilution rate showing absorbance (OD492) = 0.5 of the antibody before immunization) x 100 Specificity: Cross rate with other antigens in the case that the ELISA value of the antibody prepared to the antigen is 100 | | |

**Table 7**

| Specificity of Paracoccus denitrificans (14907) antiserum | | |
|---|---|---|
| Name of bacterium | ELISA value | Specificity (%) |
| Agrobacterium radiobacter | 72.61 | 6.990 |
| Alcaligenes denitrificans | 11.31 | 1.089 |
| Bacillus licheniformis | 5.080 | 0.489 |
| Flavobacterium sp. | 18.48 | 1.779 |
| Flavobacterium sp. | 8.100 | 0.780 |
| Paracoccus denitrificans | 1039 | 100.0 |
| Pseudomonas stutzeri | 116.7 | 11.23 |
| Pseudomonas fluorescens | 24.55 | 2.363 |
| Pseudomonas picketti | 62.88 | 6.053 |
| Thiobacillus denitrificans | 8.220 | 0.791 |
| ELISA value: (Dilution rate showing absorbance (OD492) = 0.5 of an antibody prepared to an antigen/dilution rate showing absorbance (OD492) = 0.5 of the antibody before immunization) x 100 Specificity: Cross rate with other antigens in the case that the ELISA value of the antibody prepared to the antigen is 100 | | |

**Table 8**

| Specificity of Pseudomonas stutzeri (14165T) antiserum | | |
|---|---|---|
| Name of bacterium | ELISA value | Specificity (%) |
| Agrobacterium radiobacter | 28.49 | 20.53 |
| Alcaligenes denitrificans | 1.250 | 0.901 |
| Bacillus licheniformis | 1.420 | 1.023 |
| Flavobacterium sp. | 7.740 | 5.576 |
| Flavobacterium sp. | 13.03 | 9.388 |
| Paracoccus denitrificans | 1.430 | 1.030 |
| Pseudomonas stutzeri | 138.8 | 100.0 |
| Pseudomonas fluorescens | 62.36 | 44.93 |
| Pseudomonas picketti | 9.890 | 7.125 |
| Thiobacillus denitrificans | 3.980 | 2.867 |
| ELISA value: (Dilution rate showing absorbance (OD492) = 0.5 of an antibody prepared to an antigen/dilution rate showing absorbance (OD492) = 0.5 of the antibody before immunization) x 100 Specificity: Cross rate with other antigens in the case that the ELISA value of the antibody prepared to the antigen is 100 | | |

**Table 9**

| Specificity of Pseudomonas fluorescens (14160T) antiserum | | |
|---|---|---|
| Name of bacterium | ELISA value | Specificity (%) |
| Agrobacterium radiobacter | 30.21 | 4.581 |
| Alcaligenes denitrificans | 0.380 | 0.058 |
| Bacillus licheniformis | 0.550 | 0.083 |
| Flavobacterium sp. | 7.790 | 1.181 |
| Flavobacterium sp. | 2.960 | 0.449 |
| Paracoccus denitrificans | 0.760 | 0.115 |
| Pseudomonas stutzeri | 36.66 | 5.559 |
| Pseudomonas fluorescens | 659.5 | 100.0 |
| Pseudomonas picketti | 2.210 | 0.335 |
| Thiobacillus denitrificans | 4.320 | 0.655 |
| ELISA value: (Dilution rate showing absorbance (OD492) = 0.5 of an antibody prepared to an antigen/dilution rate showing absorbance (OD492) = 0.5 of the antibody before immunization) x 100 Specificity: Cross rate with other antigens in the case that the ELISA value of the antibody prepared to the antigen is 100 | | |

**Table 10**

| Specificity of Pseudomonas picketti (JCM 5969T) antiserum | | |
|---|---|---|
| Name of bacterium | ELISA value | Specificity (%) |
| Agrobacterium radiobacter | 17.43 | 7.55 |
| Alcaligenes denitrificans | 5.530 | 2.39 |
| Bacillus licheniformis | 0.470 | 0.20 |
| Flavobacterium sp. | 14.60 | 6.32 |
| Flavobacterium sp. | 23.03 | 9.97 |
| Paracoccus denitrificans | 1.090 | 0.47 |
| Pseudomonas stutzeri | 5.220 | 2.26 |
| Pseudomonas fluorescens | 12.90 | 5.59 |
| Pseudomonas picketti | 230.9 | 100.0 |
| Thiobacillus denitrificans | 2.410 | 1.04 |
| ELISA value: (Dilution rate showing absorbance (OD492) = 0.5 of an antibody prepared to an antigen/dilution rate showing absorbance (OD492) = 0.5 of the antibody before immunization) x 100 Specificity: Cross rate with other antigens in the case that the ELISA value of the antibody prepared to the antigen is 100 | | |

**Table 11**

| Specificity of Thiobacillus denitrificans (JCM 3870) antiserum | | |
|---|---|---|
| Name of bacterium | ELISA value | Specificity (%) |
| Agrobacterium radiobacter | 34.70 | 10.10 |
| Alcaligenes denitrificans | 17.82 | 5.186 |
| Bacillus licheniformis | 1.880 | 0.547 |
| Flavobacterium sp. | 14.12 | 4.109 |
| Flavobacterium sp. | 12.67 | 3.687 |
| Paracoccus denitrificans | 4.490 | 1.307 |
| Pseudomonas stutzeri | 14.49 | 4.217 |
| Pseudomonas fluorescens | 14.45 | 4.205 |
| Pseudomonas picketti | 34.70 | 10.10 |
| Thiobacillus denitrificans | 343.6 | 100.0 |
| ELISA value: (Dilution rate showing absorbance (OD492) = 0.5 of an antibody prepared to an antigen/dilution rate showing absorbance (OD492) = 0.5 of the antibody before immunization) x 100 Specificity: Cross rate with other antigens in the case that the ELISA value of the antibody prepared to the antigen is 100 | | |

As is apparent from Table 2, Table 3, Table 4, Table 5, Table 6, Table 7, Table 8, Table 9, Table 10 and Table 11, the prepared 10 kinds of antiserums have a high specificity in almost all cases, and these antiserums with a high specificity are useful for detecting denitrifying bacteria in activated sludge, water or soil conveniently employing reaction characteristics such as an agglutination reaction to each bacterium of said antibody as a direct index, employing a coloring reaction correlative to said reaction characteristics as an index, or employing an antibody-sensitized latex for detection.

### 4. Preparation of an Antibody-Sensitized Latex

After each of the above-prepared antiserums was purified by the protein A column kit (manufactured by Amersham), it was diluted with a 0.1M glycine buffered-saline solution (pH: 8.2) so that it should have a proper protein concentration.

Into 1 volume of the antiserum was added 1 volume of a Latex H0902 solution (manufactured by Nihon Gousei Gom, JP, particle diameter: 0.98 µm, specific gravity: 1.5 g/cc); they were mixed well, and then left to stand at 37 °C for one hour so that the antibody should be adsorbed on the latex. Thereafter, a buffer containing 1 % BSA was added therein to terminate the reaction of it. After the surplus antibody was removed from it according to centrifugal and washing, the preservative solution was suspended into it so that the latex concentration of it should be 0.25 %.

### 5. Agglutination Reaction Test

The number of denitrifying bacteria was measured by the microtiter method employing an antibody-sensitized latex according to the present invention.

As a comparative control test, a sample was diluted with a 0.85 % physiological saline according to 10 grades of dilution at 1/10 concentrations employing a flat plate method and applied onto a plate prepared using an LB medium, and the measured value obtained from the number of colonies formed was deemed to be a standard value; and the measured value obtained by the MPN method according to 10 grades of dilution at 1/10 concentrations employing 5 test tubes per group was doomed to be the value of the comparative control group.

### 6. Examination of the Optimum Antibody Protein Concentration in Sensitization

The optimum antibody protein concentration in sensitization was examined. Each denitrifying bacterium antibody was examined at concentrations of an antibody protein to be used for sensitization of 0.03, 0.04, 0.05, 0.06, 0.08 and 0.1 (mg/ml). Regarding other conditions, employing H0902 as a latex with a high specific gravity, the temperature of sensitization was adjusted to 37 °C and the time of sensitization was adjusted to one hour, employing a glycine buffered-saline solution, and the concentration of a sensitized latex was adjusted to 0.25 % and the time of an agglutination reaction was adjusted to 3 to 15 hours.

Regarding the number of bacteria for comparison, since Thiobacillus denitrificans of an autotrophic bacterium is slow in proliferation, it was measured with a microscope employing a hemocytometer.

Since Agrobacterium radiobacter (IFO 13532T strain) , Alcaligenes denitrificans (IFO 15125T strain), Bacillus licheniformis (IFO 12200T strain), Flavobacterium sp. (IFO 15128 strain), Flavobacterium sp. (IFO 15147 strain), Paracoccus denitrificans (IFO 14907T strain), Pseudomonas stutzeri (IFO 14165T strain) , Pseudomonas fluorescens (IFO 14160T strain) and Pseudomonas picketti (JCM 5969T strain) of denitrifying bacteria are heterotrophic bacteria, they were measured according to the flat plate method capable of judging the number of living bacteria from the number of colonies.

A dilution series was prepared employing, as original numbers of bacteria, 1.3 x 10⁸ CFU/ml of Agrobacterium radiobacter, 2.6 x 10⁸ CFU/ml of Alcaligenes denitrificans, 4.4 x 10⁸ CFU/ml of Bacillus licheniformis, 2.5 x 10⁷ CFU/ml of Flavobacterium sp. (IFO 15128 strain), 2.5 x 10⁷ CFU/ml of Flavobacterium sp. (IFO 15147 strain), 1.4 x 10⁸ CFU/ml of Paracoccus denitrificans, 1.3 x 10⁸ CFU/ml of Pseudomonas stutzeri, 1.0 x 10⁸ CFU/ml of Pseudomonas fluorescens, 6.5 x 10⁸ CFU/ml of Pseudomonas picketti and 3.6 x 10⁷ CFU/ml of Thiobacillus denitrificans. The results are shown in Table 12.

**Table 12**

| Changes of lowest limits of quantitative values according to the concentration of antibodies | | | | | | |
|---|---|---|---|---|---|---|
| | 30 µg/ml | 40 µg/m | 150 µg/ml | 60 µg/m | 180 µg/ml | 100 µg/ml |
| Agrobacterium radiobacter | 1.0x10⁶ | 1.0x10⁶ | 1.0x10⁶ | 5.0x10⁵ | 5.0x10⁵ | 5.0x10⁵ |
| Alcaligenes denitrificans | 4.8x10⁶ | 4.8x10⁶ | 2.4x10⁶ | 2.4x10⁶ | 2.4x10⁶ | 2.4x10⁶ |
| Bacillus licheniformis | 5.0x10⁷ | 5.0x10⁷ | 2.4x10⁷ | 2.4x10⁷ | 1.2x10⁷ | 1.2x10⁷ |
| Flavobacterium sp. | 4.8x10⁷ | 4.8x10⁷ | 4.8x10⁷ | 2.4x10⁷ | 1.2x10⁷ | 1.2x10⁷ |
| Flavobacterium sp. | 4.8x10⁷ | 4.8x10⁷ | 2.4x10⁷ | 2.4x10⁷ | 1.2x10⁷ | 1.2x10⁷ |
| Paracoccus denitrificans | 9.0x10⁶ | 9.0x10⁶ | 9.0x10⁶ | 4.5x10⁶ | 4.5x10⁶ | 4.5x10⁶ |
| Pseudomonas stutzeri | 1.2x10⁷ | 1.2x10⁷ | 6.0x10⁶ | 6.0x10⁶ | 6.0x10⁶ | 6.0x10⁶ |
| Pseudomonas fluorescens | 2.0x10⁵ | 2.0x10⁵ | 2.0x10⁵ | 2.0x10⁵ | N.D | N.D |
| Pseudomonas picketti | 8.0x10⁷ | 8.0x10⁷ | 4.0x10⁷ | 4.0x10⁷ | 4.0x10⁷ | 4.0x10⁷ |
| Thiobacillus denitrificans | 4.4x10⁶ | 2.2x10⁶ | 2.2x10⁶ | 2.2x10⁶ | 2.2x10⁶ | 5.0x10⁵ |
| N.D: Not detected. | | | | | | |

As is apparent from Table 12, when denitrifying bacteria were sensitized at a concentration lower than 0.05 mg/ml, sensitivity tended to decrease. 7. Comparison between Denitrifying Bacteria Staring and Samples according to the Official Method

In order to confirm the efficiency of an antibody-sensitized latex according to the present invention, the number of bacteria in denitrifying bacteria was measured according to the microtiter method.

As a comparative control test, a sample was diluted with a 0.85 % physiological saline according to 10 grades of dilution at 1/10 concentrations employing a flat plate method and applied onto a plate prepared using an LB medium and the value obtained by measuring the number of formed colonies was deemed to be a standard value, which was compared with the measured value obtained by the MPN method according to 10 grades of dilution at 1/10 concentrations employing 5 test tubes per group. The results of comparative examination employing denitrifying bacterium strains stocked and the results of the measurement of field samples are shown in Table 13 and Table 14, respectively.

**Table 13**

| Comparison among various methods of measurement employing an Alcaligenes denitrificans antibody-sensitized latex | | | |
|---|---|---|---|
| Sample No. | Number of living bacteria (CFU/ml) | Latex measured value (CFU/ml) | Measured value by MPN method (MPN/ml) |
| NO. 1 | 2.6 x 10⁹ | 2.6 x 10⁹ | 2.4 x 10⁹ |
| No. 2 | 2.9 x 10⁸ | 1.6 x 10⁸ | 3.5 x 10⁸ |
| No. 3 | 1.3 x 10⁸ | 1.6 x 10⁸ | 3.5 x 10⁸ |
| No. 4 | 1.2 x 10⁸ | 3.2 x 10⁸ | 2.4 x 10⁸ |

**Table 14**

| Comparison between methods of quantitative determination employing an activated sludge sample | | |
|---|---|---|
| Sample No. | Microtiter method | MPN method |
| 1 | 4.0 x 10⁷ CFU/ml | 4.6 x 10³ MPN/ml |
| Sensitized latex reagent: Alcaligenes denitrificans antibody-sensitized latex | | |

As is apparent from Table 13 and Table 14, pure culture systems employing strains stocked showed a consensus of quantitative values in any of the flat plate method, the microtiter method and the MPN method. It suggests that the microtiter method employing a denitrifying bacterium antibody can perform precise quantitative determination of the number of bacteria and is excellent in convenience as compared with the official method, and that it is a method for rapid quantitative determination of it.

As a result of measuring field samples, the values obtained by quantitative determination of them according to the MPN method were slightly smaller than those of the microtiter method.

The MPN method is a system proliferating target bacteria according to culture selectively and determining the number of bacteria quantitatively, but data to be obtained become inaccurate at times. The following reasons can be guessed for this matter.
(1) The Giruti (phonetic) medium to be often employed for the quantitative determination of denitrifying bacteria supports mainly the growth of denitrifying bacteria of Pseudomonas; selectivity thereof is high but final measured values are rather low.
(2) Since nitric acid ions are employed as a substrate, denitrifying bacteria having no reductase to reduce nitric acid (e.g., Flavobacterium genus) cannot be determined quantitatively.
(3) In the case of denitrifying bacteria having no reductase to reduce nitrous oxide, nitrous oxide gas is produced as a final reduction product; however, since nitrous oxide has a large solubility and the gas cannot be stored in a Durham's tube, it cannot be judged whether it is positive of not.

Moreover, it has become clear recently that the technique monitoring the quantity of bacteria by culturing them shows values 1 to 2 orders lower than the actual quantity. According to the above, the reasons for the occurrence of a difference of 2 to 3 orders between the latex method and the MPN method in actual samples in spite of the consensus of values in the pure culture systems seem to be as follows:
(i) Since the Giruti (phonetic) medium was employed in the MPN method, the Pseudomonas became a prior species in a test tube and the proliferation of the Alcaligenes genus was restrained.
(ii) Since actual samples were subjected to quantitative determination depending on culture, the values of quantitative determination of them became lower. Therefore, it is deemed to be very important to develop a method of quantitative determination of them employing an antibody-sensitized latex comprising an antibody specific for denitrifying bacteria, which does not depend on the proliferation of the bacteria.

However, it can be confirmed that, in connection with the objects of the present development intending to establish a method of identifying target bacteria having an activity (to produce nitrogen gas and nitrous oxide gas due to the reduction of nitric acid and nitrous acid) in activated sludge, water or soil and controlling the number of bacteria rapidly and precisely, the microtiter method employing antibody-sensitized latex particles for detecting denitrifying bacteria according to the present invention is a superior method and can be employed for the above objects sufficiently.

### Industrial Applicability

As described in detail above, the present invention relates to an antibody-sensitized latex for detecting denitrifying bacteria comprising latex particles and an antibody, wherein the antibody recognizes denitrifying bacteria specifically and is adsorbed on the latex particles, and it becomes possible by using said antibody-sensitized latex to detect and determine denitrifying bacteria easily and conveniently, while a conventional method for detecting them has required a long period of time and technical expertise.

Moreover, the present invention makes it possible to identify target bacteria having an activity (to produce nitrogen gas and nitrous oxide gas due to the reduction of nitric acid and nitrous acid) in activated sludge, water or soil by preparing antibodies specific for denitrifying bacteria and employing one or optionally plural kinds thereof and to measure the number of bacteria rapidly and precisely.

## Claims

1. An antibody-sensitized latex for denitrifying bacteria, characterized by comprising latex particles and an antibody, wherein the antibody recognizes denitrifying bacteria specifically and is adsorbed on the latex particles.

2. The antibody-sensitized latex for denitrifying bacteria as claimed in Claim 1, wherein said latex particles are latex particles having a high specific gravity of about 1.5 g/cc.

3. The antibody-sensitized latex for denitrifying bacteria as claimed in Claim 1 or 2, wherein said latex particles are latex particles having an average particle diameter of about 1.0 µm.

4. A method of detecting denitrifying bacteria, characterized by employing the antibody-sensitized latex for denitrifying bacteria as claimed in Claim 1, 2 or 3.
